# EUROPEAN PATENT SPECIFICATION

(11) **EP 2 922 212 B1**
(45) Date of publication and mention of the grant of the patent: **21.08.2019**
(21) Application number: 15159217.7
(22) Date of filing: 16.03.2015
(51) Int. Cl.: H04B 1/3827, H04W 52/36, H04M 1/725

(54) **METHOD, APPARATUS AND SYSTEM FOR CONTROLLING EMISSION**
VERFAHREN, VORRICHTUNG UND SYSTEM ZUR REGULIERUNG VON EMISSIONEN
PROCÉDÉ, APPAREIL ET SYSTÈME POUR CONTRÔLER UNE ÉMISSION

(30) Priority: 20.03.2014 CN 201410106272
(43) Date of publication of application: 23.09.2015
(73) Proprietor: Xiaomi Inc., Beijing 100085 (CN)
(72) Inventor: Gao, Yuan, 100085 Beijing (CN); Pi, Xiaohui, 100085 Beijing (CN); Jiao, Xiaogang, 100085 Beijing (CN)
(74) Representative: Sadler, Peter Frederick

(56) References cited:
- EP-A1- 2 670 054
- CN-A- 102 984 359
- US-A1- 2011 309 941
- US-A1- 2012 257 657

## Description

### TECHNICAL FIELD

The present disclosure generally relates to the field of Internet terminals, and more particularly, to a method, an apparatus and a system for controlling emission.

### BACKGROUND

Terminals such as smart phones, tablet PCs and e-book readers usually adopt electromagnetic waves to achieve wireless transfer of information, these electromagnetic waves are also known as mobile phone radiation.

In order to guarantee better communication quality, a mobile phone usually emits electromagnetic waves that achieve an optimal signal. The mobile phone will use as large an emission power as possible to guarantee the strongest signal, and consequently the mobile phone radiation will reach a maximum value as well. Although the potential harm of mobile phone radiation to humans is controversial, many groups of people, such as the elderly, children, patients and pregnant women limit or avoid mobile phone use to be safe.

The inventors have appreciated that the terminals mentioned above default to optimizing their signals, which entails a larger emission strength, thereby generating greater radiation, which is not conducive to daily use for many groups of people.

US 2012/0257657 describes the dynamic control of electromagnetic radiation emissions from wireless devices using variable transmit power limits acquired through RFID/NFC tags that indicate power limits. The power limiting tags are embedded in clothing, furniture etc. US2011/309941 and EP2670054 are further examples of electromagnetic radiation monitor and control devices according to the state of the art. CN 102984359 describes a method of automatically setting-up mobile phone parameters. A plurality of types of the set-up of mobile phone parameters are preset in a mobile phone; the mobile phone confirms which type a mobile phone user is matched with according to information provided by the mobile phone user; and the mobile phone initiates the set-up of the mobile phone parameters of a corresponding type according to confirmation results. The user is automatically identified, and the appropriate types of parameters are set by pre-recorded information, the automatic identification including facial recognition, voice recognition, fingerprint recognition, iris recognition, or odor identification.

### SUMMARY

The invention is defined in the independent claims to which reference is now directed. Preferred features are set out in the dependent claims.

In order to overcome the problems existing in the relevant technologies, the present disclosure provides a method, an apparatus and a system for controlling emission. The technical solutions are as follows.

According to a first aspect of embodiments of the present disclosure, a method is provided for controlling radiation emission. The method is used in a radiation emitting terminal that emits radiation in use and includes, at the terminal:
acquiring a user type of a current user; and
if the user type of the current user is a predetermined user type for which radiation exposure should be reduced, controlling the radiation emitting terminal to reduce a default emission power.

Acquiring the user type of the current user includes:
collecting biological parameters of the current user; and determining the user type of the current user according to the biological parameters;
   or,
receiving biological parameters sent from a wearable apparatus, wherein the biological parameters are sent after the wearable apparatus collects the biological parameters of the current user; and determining the user type of the current user according to the biological parameters;
   or,
receiving a type signal sent from the wearable apparatus; and acquiring the user type of the current user according to the type signal, wherein the type signal is sent after the wearable apparatus collects the biological parameters of the current user and the user type of the current type is determined according to the biological parameters.

The biological parameters include at least one of a heartbeat, a pulse, a sleep record, a mood record, a motion record, a temperature record and a calorie intake record of a user.

Controlling the radiation emitting terminal to reduce its own emission power may include:
if the radiation emitting terminal is in an active, or communication, state, controlling the radiation emitting terminal to determine the emission power with a condition of satisfying a basic communication quality; and
if the radiation emitting terminal is in a standby state, controlling the radiation emitting terminal to determine the emission state with a condition of a minimum radiation value.

The method may further include:
if the radiation emitting terminal is in the communication state and the communication state is providing voice communication, optimizing the voice communication in a predetermined manner, wherein the predetermined manner includes at least one of increasing an audio signal gain, increasing a volume and noise reduction, or denoising.

According to a second aspect of embodiments of the present disclosure, there is provided a radiation emitting terminal that uses electromagnetic waves to wirelessly transfer data, the terminal including:
a type determining module configured to acquire a user type of a current user; and
a radiation reducing module configured to, determine when the user type of the current user is a user type for which for which radiation exposure is to be reduced exposure should be reduced and, if so, to control the radiation emitting terminal to reduce a default emission power.

The type determining module includes:
a parameter collecting unit configured to collect biological parameters of the current user; and a type determining unit configured to determine the user type of the current user according to the biological parameters;
   or,
a parameter receiving unit configured to receive biological parameters sent from a wearable apparatus, wherein the biological parameters are sent after the wearable apparatus collects the biological parameters of the current user; and the type determining unit being configured to determine the user type of the current user according to the biological parameters;
   or,
a type receiving unit configured to receive a type signal sent from the wearable apparatus; and a type acquiring unit configured to acquire the user type of the current user according to the type signal, wherein the type signal is sent after the wearable apparatus collects the biological parameters of the current user and the user type of the current user is determined according to the biological parameters.

The biological parameters include at least one of a heartbeat, a pulse, a sleep record, a mood record, a motion record, a temperature record and a calorie intake record of a user.

The radiation reducing module may include a communication reducing unit, and/or, a standby reducing unit, wherein

the communication reducing unit is configured to, when the radiation emitting terminal is in a communication state, control the radiation emitting terminal to determine the emission power with a condition of satisfying a basic communication quality, and

the standby reducing unit is configured to, when the radiation emitting terminal is in a standby state, control the radiation emitting terminal to determine the emission power with a condition of a minimum radiation value.

The terminal may further include:
a voice optimizing module configured to, when the radiation emitting terminal is in a communication state and the communication state includes providing voice communication, optimize the voice communication in a predetermined manner, wherein the predetermined manner includes at least one of including an audio signal gain, increasing a volume and denoising or noise reduction.

According to a third aspect of embodiments of the present disclosure, there is provided a system for controlling radiation emission, the system includes a wearable apparatus and a radiation emitting terminal according to the second aspect, and the wearable apparatus and the radiation emitting terminal are connected to each other through a wired network or a wireless network, wherein
the wearable apparatus is configured to collect biological parameters of a current user, and send the biological parameters to the radiation emitting terminal of the current user, and
the radiation emitting terminal is configured to receive the biological parameters sent from the wearable apparatus, determine a user type of the current user according to the biological parameters, and reduce a default emission power when the user type of the current user is a user type for which for which radiation exposure is to be reduced exposure should be reduced.

According to a fourth aspect of embodiments of the present disclosure, there is provided a system for controlling radiation emission. The system includes a wearable apparatus and a radiation emitting terminal according to the second aspect, and the wearable apparatus and the radiation emitting terminal are connected to each other through a wired network or a wireless network, wherein
the wearable apparatus is configured to collect biological parameters of a current user, determine a user type of the current user according to the biological parameters, and send a type signal to the radiation emitting terminal of the current user according to the user type, and
the radiation emitting terminal is configured to receive the type signal sent from the wearable apparatus, acquire the user type of the current user according to the type signal, and reduce a default emission power when the user type of the current user is a user type for which radiation exposure should be reduced.

According to a fifth aspect of the embodiments of the present disclosure, there is provided a computer program, which when executed on a processor of a terminal, performs a method of the first aspect of the embodiments of the present disclosure.

The technical solutions provided by the embodiments of the present disclosure may include the following advantageous effects.

The user type of the current user is acquired, and if the user types of the current user are the user types for which radiation exposure needs to be reduced, the radiation emitting terminal is controlled to reduce the default emission power, thereby solving the problem of larger radiation caused by a terminal in the relevant technologies adopting a default emission mechanism of optimal signal, and achieving an effect of reducing radiation by not adopting the default emission mechanism of optimal signal for the user types for which radiation exposure is to be reduced.

It shall be understood that both the foregoing general description and the following detailed description are exemplary and explanatory only and may not limit the present disclosure.

### BRIEF DESCRIPTION OF THE DRAWINGS

The accompanying drawings which are hereby incorporated in and constitute a part of this specification, illustrate embodiments consistent with the disclosure, and together with the description, serve to explain the principles of the disclosure.
Fig. 1 is a flow diagram showing a method for controlling emission, according to an exemplary embodiment;
Fig. 2 is a flow diagram showing another method for controlling emission, according to an exemplary embodiment;
Fig. 3 is a flow diagram showing still another method for controlling emission, according to an exemplary embodiment;
Fig. 4 is a flow diagram showing yet still another method for controlling emission, according to an exemplary embodiment;
Fig. 5 is a flow diagram showing yet still another method for controlling emission, according to an exemplary embodiment;
Fig. 6 is a diagram showing an apparatus for controlling emission, according to an exemplary embodiment;
Fig. 7A is a diagram showing another apparatus for controlling emission, according to an exemplary embodiment;
Figs. 7B-7E are diagrams of four different implementations of a type determining module shown in Fig. 7A;
Fig. 8 is a diagram showing an apparatus for controlling emission, according to an exemplary embodiment; and
Fig. 9 is a structural block diagram showing a radiation emitting terminal, according to an exemplary embodiment.

### DETAILED DESCRIPTION

Detailed description of the exemplary embodiments will be made herein, with examples thereof to be shown in the accompanying drawings. In the following descriptions, when reference is made to the accompanying drawings, unless expressed otherwise, the same number in different drawings refers to the same or similar elements. The following described exemplary embodiments do not represent all embodiments that are consistent with the present disclosure. On the contrary, they are only examples of the devices and the methods that are consistent with some of the aspects of the present disclosure as recited in the claims.

"Radiation emitting terminal" mentioned herein includes a mobile phone, a tablet PC, a router, an e-book reader, a MP3 (Moving Picture Experts Group Audio Layer III) player, a MP4 (Moving Picture Experts Group Audio Layer IV) player, a portable laptop computer, a desktop computer and the like.

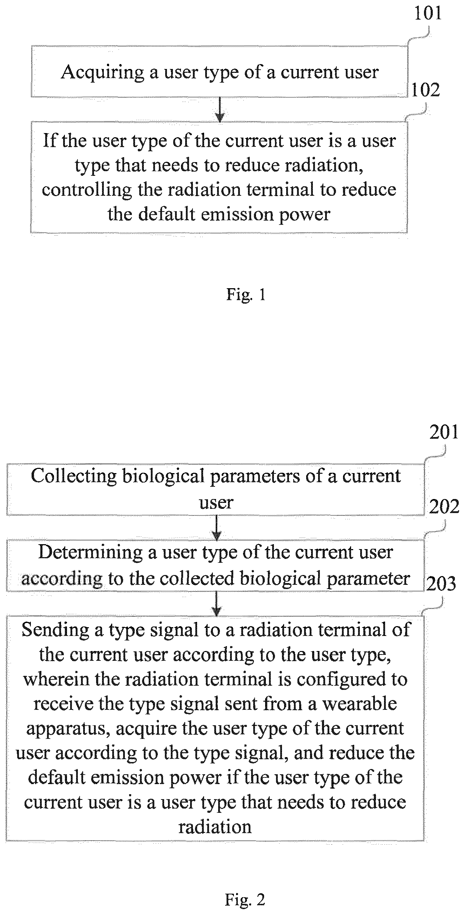

Fig. 1 is a flow diagram showing a method for controlling emission, according to an exemplary embodiment. As shown in Fig. 1, the method for controlling emission is used in a radiation emitting terminal and includes the following steps.

In step S101, user types of a current user are acquired.

The user types may be classified into ordinary people, the elderly, children or pregnant women, wherein the elderly, the children and the pregnant women are the user types for which radiation exposure is to be reduced. The user types may also be classified into healthy people, sub-healthy people or patients, wherein the sub-healthy people and the patients are the user types for which radiation exposure is to be reduced.

The user types are acquired based on biological parameters of the user.

In step S102, if the user types of the current user are the user types for which radiation exposure is to be reduced, the radiation emitting terminal is controlled to reduce the default emission power.

When a default emission strategy is a signal priority strategy, the default emission power usually is an emission power having the largest radiation emission.

In conclusion, in the method for controlling emission provided by the present embodiment, the user types of the current user are acquired, and if the user types of the current user are the user types for which radiation exposure is to be reduced, the radiation emitting terminal is controlled to reduce the default emission power, thereby solving the problem of larger radiation emission caused by a terminal in the relevant technologies adopting a default emission mechanism that optimises signal, and achieving the effect of reducing radiation emission by not adopting the default emission mechanism for optimal signal for the user types for which radiation exposure is to be reduced.

Fig. 2 is a flow diagram showing another method for controlling emission, according to an exemplary embodiment. As shown in Fig. 2, the method for controlling emission is used in a wearable apparatus and includes the following steps.

In step S201, biological parameters of a current user are collected.

The biological parameters include at least one of a heartbeat, a pulse, a sleep record, a mood record, a motion record, a temperature record and a calorie intake record of a user.

In step S202, a user type of the current user is determined according to the collected biological parameters.

In step S203, a type signal is sent to a radiation emitting terminal of the current user according to the user type, and the radiation emitting terminal is configured to receive the type signal sent from the wearable apparatus, acquire the user type of the current user according to the type signal, and reduce the default emission power if the user type of the current user is a user type for which radiation exposure is to be reduced.

In conclusion, in the method for controlling emission provided by the present embodiment, the user type of the current user is acquired, and if the user type of the current user is the user type for which radiation exposure is to be reduced, the radiation emitting terminal is controlled to reduce the default emission power, thereby solving the problem of larger radiation caused by that a terminal in the relevant technologies adopts a default emission mechanism of optimal signal, and achieving an effect of reducing radiation by not adopting the default emission mechanism of optimal signal for the user type for which radiation exposure is to be reduced.

Since the radiation emitting terminal may acquire the user type in various kinds of manners, for example, the radiation emitting terminal may acquire the defined user type of the current user by means of input manners such as a voice form, a physical keyboard, a virtual keyboard, a touch signal or a shake gesture. For another example, if the radiation emitting terminal is a terminal such as a mobile phone and a tablet PC that is used by the user in daily life in a contact way, the radiation emitting terminal may acquire the user type based on the biological parameters of the current user. For another example, if the radiation emitting terminal is a terminal such as a mobile phone and a tablet PC that is used by the user in daily life in a non-contact way, the radiation emitting terminal may acquire the user type based on the biological parameters of the current user by means of the wearable apparatus. Two cases in which the user type is acquired based on the biological parameters are respectively described through different embodiments as follows.

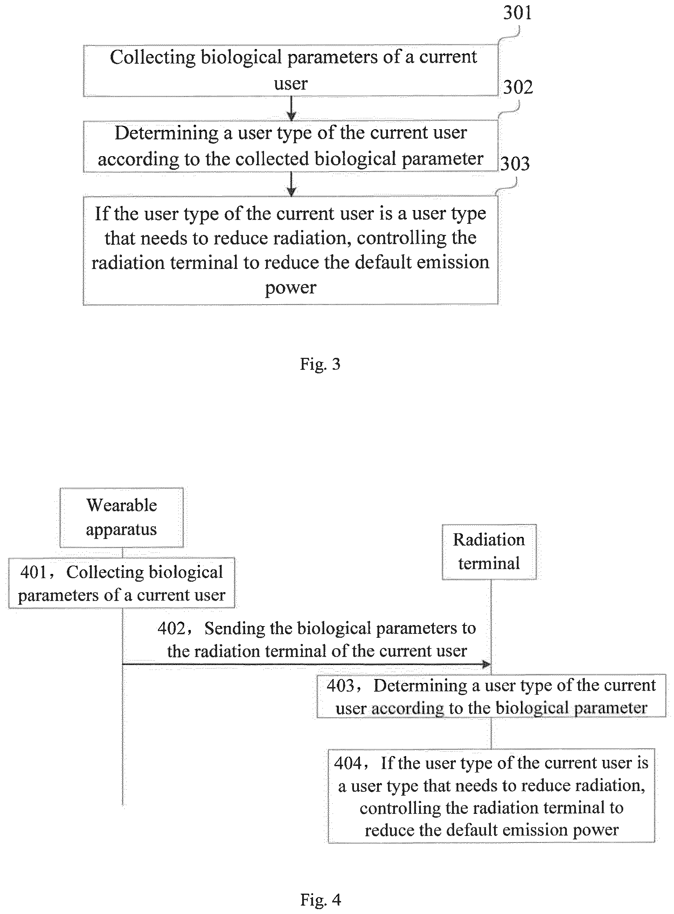

Fig. 3 is a flow diagram showing still another method for controlling emission, according to an exemplary embodiment. As shown in Fig. 3, the method for controlling emission is used in a radiation emitting terminal, and a user type is acquired by the radiation emitting terminal based on biological parameters of a current user, the method includes the following steps.

In step S301, the biological parameters of the current user are collected.

The biological parameters include at least one of a heartbeat, a pulse, a sleep record, a mood record, a motion record, a temperature record and a calorie intake record of a user.

The radiation emitting terminal acquires the mood record and the calorie intake record of the user by an input from the user. The radiation emitting terminal monitors and analyses the sleep record, the mood record and the motion record of the user by a triaxial accelerometer. The radiation emitting terminal acquires the user's state of mind and pulse by an electrode and by a principle of an electrocardiogram. The radiation emitting terminal acquires the temperature of the user by a temperature sensor.

In step S302, the user type of the current user is determined according to the biological parameters.

After acquiring the biological parameters of the current user, the radiation emitting terminal may query the user type of the current user in a predetermined corresponding relationship, according to a value of one biological parameter or values of several combined biological parameters. The predetermined corresponding relationship includes a corresponding relationship between different values of the biological parameters and different user types.

That is, after the biological parameters of the current user are collected, the radiation emitting terminal may query the user type that matches the value of the biological parameters in the predetermined corresponding relationship, according to the value of the biological parameters.

In step S303, if the user type of the current user is a user type for which radiation exposure is to be reduced, the radiation emitting terminal is controlled to reduce the default emission power.

The user type for which radiation exposure is to be reduced may be the elderly, children or pregnant women. When the user type of the current user is the user type for which radiation exposure is to be reduced, the radiation emitting terminal is controlled to reduce the default emission power.

If the radiation emitting terminal is in a communication state, the radiation emitting terminal is controlled to determine an emission power with a condition of satisfying a basic communication quality. The satisfying the basic communication quality refers to that signal intensity only may maintain the basic communication quality rather than an optimal communication quality. On premise that the basic communication quality is maintained, the emission power is reduced as much as possible. This process may be realized by an empirical threshold. When the signal intensity is lower than the empirical threshold, the emission power is increased, and when the signal intensity is higher than the empirical threshold, the emission power is reduced.

If the radiation emitting terminal is in a standby state, the radiation emitting terminal is controlled to determine the emission power with a condition of a minimum radiation value. For example, if a mobile phone is in the standby state, the mobile phone is controlled to determine the emission power with the condition of the minimum radiation value.

It needs to be further explained that, if the radiation emitting terminal is in the communication state and the communication state is a voice communication, the voice communication is optimized by a predetermined manner, and the predetermined manner includes at least one of increasing an audio signal gain, increasing a volume and denoising.

In conclusion, in the method for controlling emission provided by the present embodiment, the user type of the current user is determined by the biological parameters of the current user, and if the user type of the current user is the user type for which radiation exposure is to be reduced, the radiation emitting terminal is controlled to reduce the default emission power, thereby solving the problem of larger radiation caused by that a terminal in the relevant technologies adopts a default emission mechanism of optimal signal, and achieving an effect of reducing radiation by not adopting the default emission mechanism of optimal signal for the user type for which radiation exposure is to be reduced.

The emission power is also determined with the condition of satisfying a basic communication quality condition when the terminal is in the communication state, such that not only can the normal use of the radiation emitting terminal be guarantied but also the emitted radiation may be reduced as much as possible.

The emission power is also determined with the condition of a minimum radiation value when the terminal is in the standby state, such that the radiation emitting terminal keeps the emitted radiation levels to a minimum value in the standby state.

When the communication state provides voice communication, some optimizations except for signal intensity are performed on the voice communication, such that the basic communication quality may be better guarantied.

Fig. 4 is a flow diagram showing yet still another method for controlling radiation emission, according to an exemplary embodiment. As shown in Fig. 4, the method for controlling emission is used in a radiation emitting terminal and a wearable apparatus. Biological parameters are collected by the wearable apparatus, the radiation emitting terminal determines a user type based on biological parameters of a current user, and the method includes the following steps.

In step S401, the wearable apparatus collects the biological parameters of the current user.

The biological parameters include at least one of a heartbeat, a pulse, a sleep record, a mood record, a motion record, a temperature record and a calorie intake record of a user.

The wearable apparatus may be a smart bracelet or a smart watch or the like. The wearable apparatus may acquire the mood record and/or the calorie intake record of the user by an input from the user. The wearable apparatus may monitor and analyse the sleep record, the mood record and/or the motion record of the user by a triaxial accelerometer. The wearable apparatus may acquire the user's state of mind and/or pulse by an electrode and by the principle of an electrocardiogram. The wearable apparatus may acquire the temperature of the user by a temperature sensor.

In step S402, the wearable apparatus sends the biological parameters to the radiation emitting terminal of the current user.

The wearable apparatus and the radiation emitting terminal of the same user are usually connected to each other through a wired network or a wireless network, for example, a smart bracelet and a smart phone of a user A achieve wireless connection through Bluetooth technology or WIFI network.

After collecting the biological parameters of the current user, the wearable apparatus sends the biological parameters to the radiation emitting terminal of the current user.

Correspondingly, the radiation emitting terminal receives the biological parameters sent from the wearable apparatus.

In step S403, the radiation emitting terminal determines the user type of the current user according to the biological parameters.

After acquiring the biological parameters of the current user, the radiation emitting terminal may query the user type of the current user in a predetermined corresponding relationship, according to a value of one biological parameter or values of several combined biological parameters. The predetermined corresponding relationship includes a corresponding relationship between different values of the biological parameters and different user types.

That is, after the biological parameters of the current user are received, the radiation emitting terminal may query the user type that matches the value of the biological parameters in the predetermined corresponding relationship, according to the value of the biological parameters.

In step S404, if the user type of the current user is a user type for which radiation exposure is to be reduced, the radiation emitting terminal is controlled to reduce the default emission power.

The user type for which radiation exposure is to be reduced may be the elderly, children or pregnant women. When the user type of the current user is the user type for which radiation exposure is to be reduced, the radiation emitting terminal is controlled to reduce the default emission power.

If the radiation emitting terminal is in a communication state, the radiation emitting terminal is controlled to determine an emission power by applying a condition of satisfying a basic communication quality. Satisfying the basic communication quality refers to altering the signal intensity such that it only maintains a basic communication quality rather than an optimal communication quality. On the premise that the basic communication quality is maintained, the emission power is reduced as much as possible. This process may be realized by an empirical threshold. When the signal intensity is lower than the empirical threshold, the emission power is increased, and when the signal intensity is higher than the empirical threshold, the emission power is reduced.

If the radiation emitting terminal is in a standby state, the radiation emitting terminal is controlled to determine the emission power by applying a condition of a minimum radiation value. For example, if a mobile phone is in the standby state, the mobile phone is controlled to determine the emission power that satisfies the condition of the minimum radiation value.

It needs to be further explained that, if the radiation emitting terminal is in the communication state and the communication state involves a voice communication, the voice communication is optimized by a predetermined manner, and the predetermined manner includes at least one of increasing an audio signal gain, increasing a volume and denoising.

In conclusion, in the method for controlling emission provided by the present embodiment, the user type of the current user is determined by the biological parameters of the current user, and if the user type of the current user is the user type for which radiation exposure is to be reduced, the radiation emitting terminal is controlled to reduce the default emission power, thereby solving the problem of larger radiation caused by a terminal in the relevant technologies adopting a default emission mechanism of optimal signal, and achieving the effect of reducing radiation by not adopting the default emission mechanism of optimal signal for the user type for which radiation exposure is to be reduced.

The emission power is also determined by applying the condition of satisfying the basic communication quality when it is in the communication state, such that the effect that not only the normal use of the radiation emitting terminal may be guaranteed but also the radiation may be reduced as much as possible.

The emission power is also determined in accordance with the condition of a minimum radiation value when it is in the standby state, such that the radiation emitting terminal keeps the radiation to a minimum level in the standby state.

When the communication state is voice communication, some optimizations except the signal intensity are performed on the voice communication, such that the basic communication quality may be better guarantied.

The biological parameters of the user are also collected by the wearable apparatus, inherent characteristics of the wearable apparatus are used, such that hardware conditions and computation amounts required for the radiation emitting terminal are reduced, resources of the radiation emitting terminal are saved, and endurance of the radiation emitting terminal is increased.

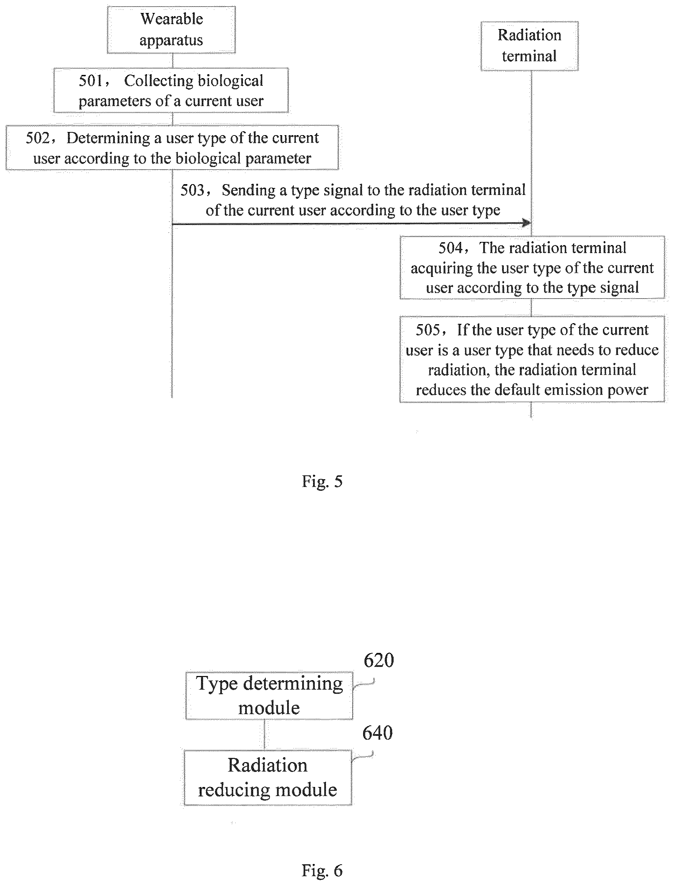

Fig. 5 is a flow diagram showing yet still another method for controlling emission, according to an exemplary embodiment. As shown in Fig. 5, the method for controlling emission is used in a radiation emitting terminal and a wearable apparatus. The wearable apparatus collects biological parameters, and determines a user type based on the biological parameters of a current user, and the method includes the following steps.

In step S501, the wearable apparatus collects the biological parameters of the current user.

The biological parameters include at least one of a heartbeat, a pulse, a sleep record, a mood record, a motion record, a temperature record and a calorie intake record of a user.

The wearable apparatus may be a smart bracelet or a smart watch or the like. The wearable apparatus acquires the mood record and the calorie intake record of the user by an input from the user. The wearable apparatus monitors and analyses the sleep record, the mood record and the motion record of the user by a triaxial accelerometer. The wearable apparatus acquires the user's state of mind and pulse by an electrode and by the principle of an electrocardiogram. The wearable apparatus acquires the temperature of the user by a temperature sensor.

In step S502, the wearable apparatus determines the user type of the current user according to the biological parameters.

After collecting the biological parameters of the current user, the wearable apparatus may query the user type of the current user in a predetermined corresponding relationship, according to a value of one biological parameter or values of several combined biological parameters. The predetermined corresponding relationship includes a corresponding relationship between different values of the biological parameters and different user types.

That is, after collecting the biological parameters of the current user, the wearable apparatus may query the user type that matches the value of the biological parameters in the predetermined corresponding relationship, according to the value of the biological parameters.

In step S503, the wearable apparatus sends a type signal to the radiation emitting terminal of the current user according to the user type.

Each user type may respectively correspond to a kind of type signal. The wearable apparatus generates the type signal of the current user according to the user type. Since the wearable apparatus and the radiation emitting terminal of the same user are usually connected to each other through a wired network or a wireless network, the wearable apparatus may send the type signal to the current user by building a connection.

Correspondingly, the radiation emitting terminal receives the type signal sent from the wearable apparatus.

It needs to be further explained that, the wearable apparatus may send the type signal to the radiation emitting terminal only when the user type of the current user is a user type for which radiation exposure is to be reduced, , although this is not a limitation on the present embodiment.

In step S504, the radiation emitting terminal acquires the user type of the current user according to the type signal.

Since each user type may respectively correspond to a kind of type signal, the radiation emitting terminal may acquire the user type of the current user according to the type signal.

In step S505, if the user type of the current user is the user type for which radiation exposure is to be reduced, the radiation emitting terminal reduces a default emission power.

The user type for which radiation exposure is to be reduced may be the elderly, children or pregnant women. When the user type of the current user is the user type for which radiation exposure is to be reduced, the radiation emitting terminal is controlled to reduce the default emission power.

If the radiation emitting terminal is in a communication state, the radiation emitting terminal is controlled to determine an emission power with a condition of satisfying a basic communication quality. Satisfying the basic communication quality may require that signal intensity is set at a level that only maintains a basic communication quality rather than an optimal communication quality. On the premise that the basic communication quality is maintained, the emission power is reduced as much as possible. This process may be realized by an empirical threshold. When the signal intensity is lower than the empirical threshold, the emission power is increased, and when the signal intensity is higher than the empirical threshold, the emission power is reduced.

If the radiation emitting terminal is in a standby state, the radiation emitting terminal is controlled to determine the emission power based on a condition of a minimum radiation value. For example, if a mobile phone is in the standby state, the mobile phone is controlled to determine the emission power that meets the condition of the minimum radiation value.

It needs to be further explained that, if the radiation emitting terminal is in the communication state and the communication state is a voice communication, the voice communication is optimized by a predetermined manner, and the predetermined manner includes at least one of increasing an audio signal gain, increasing a volume and denoising.

In conclusion, in the method for controlling emission provided by the present embodiment, the user type of the current user is determined by the biological parameters of the current user, and if the user type of the current user is the user type for which radiation exposure is to be reduced, the radiation emitting terminal is controlled to reduce the default emission power, thereby solving the problem of larger radiation caused by a terminal in the relevant technologies adopting a default emission mechanism of optimal signal, and achieving the effect of reducing radiation by not adopting the default emission mechanism for optimal signal for the user types for which radiation exposure is to be reduced.

The emission power is also determined with the condition of satisfying the basic communication quality when it is in the communication state, such that not only is the normal use of the radiation emitting terminal guarantied but also the radiation may be reduced as much as possible.

The emission power is also determined based on the condition of a minimum radiation value when it is in the standby state, such that the radiation emitting terminal keeps the radiation to a minimum level in the standby state.

When the communication state is voice communication, some optimizations except the signal intensity are performed on the voice communication, such that the basic communication quality may be better guarantied.

The wearable apparatus collects the biological parameters of the user, and determines the user type, and inherent characteristics of the wearable apparatus are used, such that hardware conditions and computation amounts required for the radiation emitting terminal are reduced, resources of the radiation emitting terminal are saved, and endurance of the radiation emitting terminal is increased.

Fig. 6 is a diagram showing an apparatus for controlling emission, according to an exemplary embodiment. Referring to Fig. 6, the apparatus includes a type determining module 620 and a radiation reducing module 640.

The type determining module 620 is configured to acquire a user type of a current user.

The radiation reducing module 640 is configured to, when the user type of the current user is a user type for which radiation exposure is to be reduced, control a radiation emitting terminal to reduce the default emission power.

In conclusion, in the apparatus for controlling emission provided by the present embodiment, the user type of the current user is determined by the biological parameters of the current user, and if the user type of the current user is the user type for which radiation exposure is to be reduced, the radiation emitting terminal is controlled to reduce the default emission power, thereby solving the problem of larger radiation caused by a terminal in the relevant technologies adopting a default emission mechanism of optimal signal, and achieving the effect of reducing radiation by not adopting the default emission mechanism of optimal signal for the user type for which radiation exposure is to be reduced.

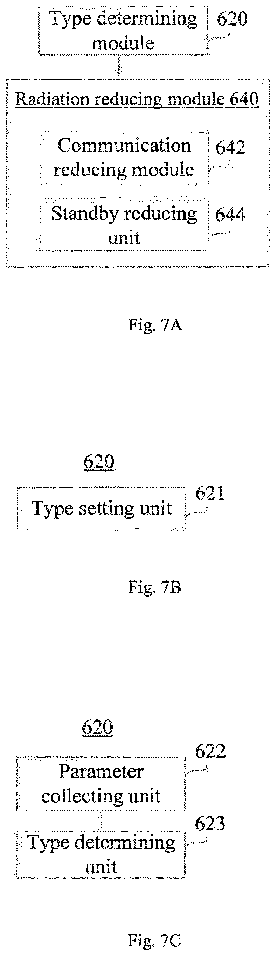

Fig. 7A is a diagram showing another apparatus for controlling emission, according to an exemplary embodiment. Referring to Fig. 7A, the apparatus may be implemented to be all or a portion of a radiation emitting terminal by software, hardware or combination thereof. The apparatus includes a type determining module 620 and a radiation reducing module 640.

The type determining module 620 is configured to acquire a user type of a current user.

The radiation reducing module 640 is configured to, when the user type of the current user is a user type for which radiation exposure is to be reduced, control the radiation module to reduce the default emission power.

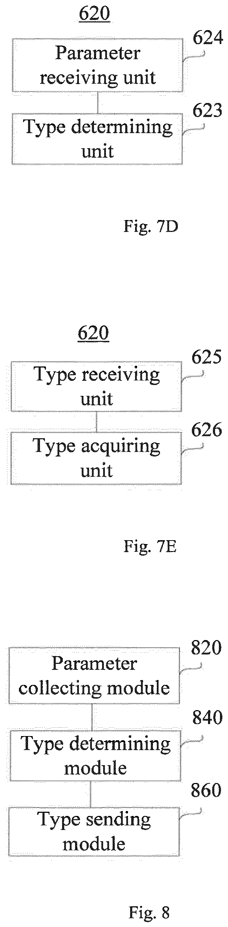

The type determining module 620 includes:
a parameter collecting unit 622 configured to collect biological parameters of the current user; and a type determining unit 623 configured to determine the user type of the current user according to the biological parameters, as shown in Fig. 7C;
   or,
a parameter receiving unit 624 configured to receive biological parameters sent from a wearable apparatus, and the biological parameters are sent after the wearable apparatus collects the biological parameters of the current user, and the type determining unit 623 being configured to determine the user type of the current user according to the biological parameters, as shown in Fig. 7D;
   or,
a type receiving unit 625 configured to receive a type signal sent from the wearable apparatus; and a type acquiring unit 626 configured to acquire the user type of the current user according to the type signal, wherein the type signal is sent after the wearable apparatus collects the biological parameters of the current user and the user type of the current user is determined according to the biological parameters, as shown in Fig. 7E.

The radiation reducing module 640 includes a communication reducing unit 642, and/or, a standby reducing unit 644,
the communication reducing unit 642 is configured to, when the radiation emitting terminal is in a communication state, control the radiation emitting terminal to determine the emission power with the condition of satisfying a basic communication quality, and
the standby reducing unit 644 is configured to, when the radiation emitting terminal is in a standby state, control the radiation emitting terminal to determine the emission power with the condition of a minimum radiation value.

Alternatively, the apparatus further includes:
a voice optimizing module 660 configured to, when the radiation emitting terminal is in the communication state and is a voice communication, optimize the voice communication in a predetermined manner, the predetermined manner including at least one of increasing an audio signal gain, increasing a volume and denoising.

In conclusion, in the apparatus for controlling emission provided by the present embodiment, the user type of the current user is determined by the biological parameters of the current user, and if the user type of the current user is the user type for which radiation exposure is to be reduced, the radiation emitting terminal is controlled to reduce the default emission power, thereby solving the problem of larger radiation caused by a terminal in the relevant technologies adopting a default emission mechanism of optimal signal, and achieving the effect of reducing radiation by not adopting the default emission mechanism of optimal signal for the user type for which radiation exposure is to be reduced.

The emission power is also determined with the condition of satisfying the basic communication quality when it is in the communication state, such that not only the normal use of the radiation emitting terminal may be guarantied but also the radiation may be reduced as much as possible.

The emission power is also determined with the condition of the minimum radiation value when it is in the standby state, such that the radiation emitting terminal keeps the radiation minimum in the standby state.

When the communication state is the voice communication, some optimizations except the signal intensity are performed on the voice communication, such that the basic communication quality may be better guarantied.

The wearable apparatus collects the biological parameters of the user, and determines the user type, and inherent characteristics of the wearable apparatus are used, such that hardware conditions and computation amounts required for the radiation emitting terminal are reduced, resources of the radiation emitting terminal are saved, and endurance of the radiation emitting terminal is increased.

Fig. 8 is a diagram showing an apparatus for controlling emission, according to an exemplary embodiment. Referring to Fig. 8, the apparatus may be implemented to be all or a portion of a wearable apparatus by software, hardware or combination thereof. The apparatus includes a parameter collecting module 820, a type determining module 840 and a type sending module 860.

The parameter collecting module 820 is configured to collect biological parameters of a current user.

The type determining module 840 is configured to determine a user type of the current user according to the collected biological parameters.

The type sending module 860 is configured to send a type signal to a radiation emitting terminal of the current user according to the user type, and the radiation emitting terminal is configured to receive a type signal of the wearable apparatus, acquire the user type of the current user according to the type signal, and reduce the default emission power if the user type of the current user is a user type for which radiation exposure is to be reduced.

With respect to the devices in the above embodiments, the specific manners for performing operations for individual modules therein have been described in detail in the embodiments regarding to the methods for controlling emission, which will not be elaborated herein.

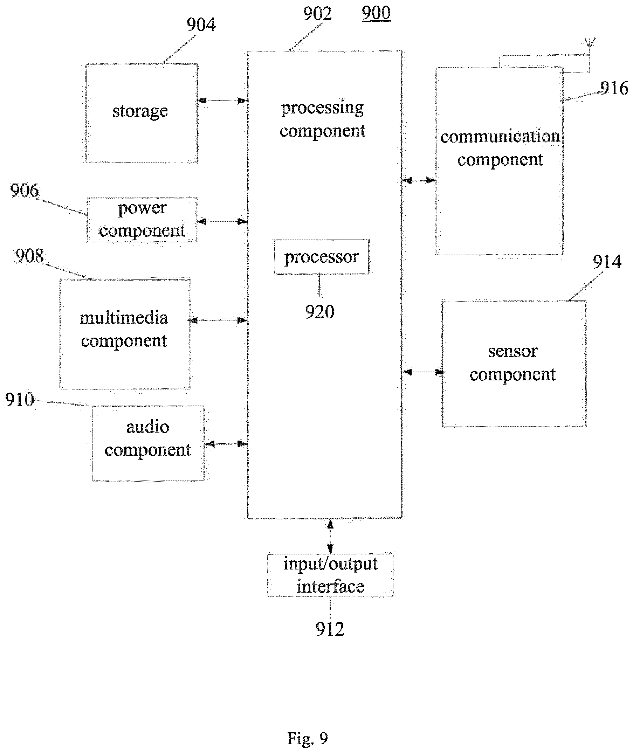

Fig. 9 is a block diagram showing a radiation emitting terminal 900, according to an exemplary embodiment. For example, the radiation emitting terminal 900 may be a mobile phone, a computer, a digital broadcast terminal, a messaging device, a gaming console, a tablet device, a medical device, exercise equipment, a personal digital assistant, a router and the like.

Referring to Fig. 9, the radiation emitting terminal 900 may include one or more of the following components: a processing component 902, a storage 904, a power component 906, a multimedia component 908, an audio component 910, an input/output (I/O) interface 912, a sensor component 914, and a communication component 916.

The processing component 902 usually controls the overall operations of the radiation emitting terminal 900, such as the operations associated with display, telephone calls, data communications, camera operations, and recording operations. The processing component 902 may include one or more processors 920 to execute instructions to perform all or a part of the steps in the above described methods. Moreover, the processing component 902 may include one or more modules which facilitate the interaction between the processing component 902 and other components. For instance, the processing component 902 may include a multimedia module to facilitate the interaction between the multimedia component 908 and the processing component 902.

The storage 904 is configured to store various types of data to support the operations of the radiation emitting terminal 900. Examples of such data include instructions for any application or method operated on the radiation emitting terminal 900, contact data, phonebook data, messages, pictures, videos, etc. The storage 904 may be implemented by using any type of volatile or non-volatile memory devices or combination thereof, such as a static random access memory (SRAM), an electrically erasable programmable read-only memory (EEPROM), an erasable programmable read-only memory (EPROM), a programmable read-only memory (PROM), a read-only memory (ROM), a magnetic memory, a flash memory, a magnetic disk or an optical disk.

The power component 906 provides power to the respective components of the radiation emitting terminal 900. The power component 906 may include a power management system, one or more power sources, and components associated with the generation, management, and distribution of power for the radiation emitting terminal 900.

The multimedia component 908 includes a screen providing an output interface between the radiation emitting terminal 900 and the user. In some embodiments, the screen may include a liquid crystal display (LCD) and a touch panel (TP). If the screen includes the touch panel, the screen may be implemented as a touch screen to receive input signals from the user. The touch panel includes one or more touch sensors to sense touches, swipes, and gestures on the touch panel. The touch sensors may not only sense a boundary of a touch or swipe action, but also sense a duration of time and a pressure associated with the touch or swipe action. In some embodiments, the multimedia component 908 includes a front camera and/or a rear camera. The front camera and/or the rear camera may receive external multimedia data while the radiation emitting terminal 900 is in an operation mode such as a photographing mode or a video mode. Each of the front camera and the rear camera may be a fixed optical lens system or have focus and optical zoom capability.

The audio component 910 is configured to output and/or input audio signals. For example, the audio component 910 includes a microphone (MIC) configured to receive external audio signals when the radiation emitting terminal 900 is in an operation mode such as a call mode, a recording mode and a voice recognition mode. The received audio signal may be further stored in the memory 904 or transmitted via the communication component 916. In some embodiments, the audio component 910 further includes a speaker to output audio signals.

The I/O interface 912 provides an interface between the processing component 902 and peripheral interface modules, such as a keyboard, a click wheel, a button, and the like. The button may include, but not limited to, a home page button, a volume button, a starting button, and a locking button.

The sensor component 914 includes one or more sensors to provide status assessments of respective aspects of the radiation emitting terminal 900. For example, the sensor component 914 may detect an open/closed status of the radiation emitting terminal 900, relative positioning of components, for example, the display and the keyboard of the radiation emitting terminal 900, a position change of the radiation emitting terminal 900 or of a component of the radiation emitting terminal 900, a presence or absence of a user contacting with the radiation emitting terminal 900, an orientation or an acceleration/deceleration of the radiation emitting terminal 900, and a temperature change of the radiation emitting terminal 900. The sensor component 914 may include a proximity sensor configured to detect the presence of nearby objects without any physical contact. The sensor component 914 may also include a light sensor such as a CMOS or CCD image sensor for use in imaging applications. In some embodiments, the sensor component 914 may also include an accelerometer sensor, a gyroscope sensor, a magnetic sensor, a pressure sensor, or a temperature sensor.

The communication component 916 is configured to facilitate communication, by wire or wirelessly, between the radiation emitting terminal 900 and other devices. The radiation emitting terminal 900 may access a wireless network based on a communication standard, such as WiFi, 2G, or 3G, or a combination thereof. In one exemplary embodiment, the communication component 916 receives a broadcast signal or broadcast associated information from an external broadcast management system via a broadcast channel. In one exemplary embodiment, the communication component 916 may further include a near field communication (NFC) module to facilitate short-range communications. For example, the NFC module may be implemented based on a radio frequency identification (RFID) technology, an infrared data association (IrDA) technology, an ultra-wideband (UWB) technology, a Bluetooth (BT) technology, and other technologies.

In exemplary embodiments, the radiation emitting terminal 900 may be implemented with one or more application specific integrated circuits (ASICs), digital signal processors (DSPs), digital signal processing devices (DSPDs), programmable logic devices (PLDs), field programmable gate arrays (FPGAs), controllers, micro-controllers, microprocessors, or other electronic components, for performing the above described methods.

In exemplary embodiments, there is also provided a non-transitory computer readable storage medium including instructions, such as the storage 904includ including the instructions executable by the processor 920 in the radiation emitting terminal 900, for performing the above-described methods. For example, the non-transitory computer-readable storage medium may be a ROM, a RAM, a CD-ROM, a magnetic tape, a floppy disc, an optical data storage device, and the like.

A non-transitory computer readable storage medium, when instructions in the storage medium are executed by a processor of a radiation emitting terminal, the radiation emitting terminal may execute a method for controlling emission, the method includes:
acquiring a user type of a current user; and
if the user type of the current user is a user type for which radiation exposure is to be reduced, controlling the radiation emitting terminal to reduce the default emission power.
Acquiring the user type of the current user may include:
collecting biological parameters of the current user; and determining the user type of the current user according to the biological parameters;
   or,
receiving biological parameters sent from a wearable apparatus, wherein the biological parameters are sent after the wearable apparatus collects the biological parameters of the current user; and determining the user type of the current user according to the biological parameters;
   or,
receiving a type signal sent from the wearable apparatus; and acquiring the user type of the current user according to the type signal, wherein the type signal is sent after the wearable apparatus collects the biological parameters of the current user and the user type of the current type is determined according to the biological parameters.

Controlling the radiation emitting terminal to reduce its own emission power may include:
if the radiation emitting terminal is in a communication state, controlling the radiation emitting terminal to determine the emission power with the condition of satisfying a basic communication quality; and
if the radiation emitting terminal is in a standby state, controlling the radiation emitting terminal to determine the emission state with the condition of a minimum radiation value.

The method may further include:
if the radiation emitting terminal is in the communication state and the communication state is a voice communication, the voice communication is optimized by a predetermined manner, and the predetermined manner includes at least one of increasing an audio signal gain, increasing a volume and denoising.

Moreover, the respective components in Fig. 9 may be recombined or deleted to be implemented as a wearable apparatus, and the wearable apparatus may be a smart phone, a smart watch and a smart glasses or the like.

A non-transitory computer readable storage medium may be provided, wherein when instructions in the storage medium are executed by a processor of the wearable apparatus, the wearable apparatus may execute a method for controlling emission, and the method includes:
collecting biological parameters of a current user;
determining a user type of the current user according to the collected biological parameters; and
sending a type signal to a radiation emitting terminal of the current user according to the user type, wherein the radiation emitting terminal is configured to receive the type signal sent from the wearable apparatus, acquire the user type of the current user according to the type signal, and reduce the default emission power, if the user type of the current user is a user type for which radiation exposure is to be reduced.

After considering this description and carrying out the embodiments disclosed herein, those skilled in the art may easily anticipate other implementation aspects of the present disclosure. The present disclosure is meant to cover any variations, usage or adaptive change of these embodiments, and these variations, usage or adaptive change follow general concept of the present disclosure and include the common knowledge or the customary technical means in the technical field that is not disclosed in the present disclosure. The description and embodiments are only exemplary, and the real range and spirit of the present disclosure are defined by the following claims.

It should be understood that the present disclosure is not limited to precise structures that are described above and shown in the accompanying drawings, and may be modified and changed without departing from the range of the present disclosure. The scope of the present disclosure is only defined by the appended claims.

## Claims

1. A method for controlling radiation emission from a radiation emitting terminal that uses electromagnetic waves to wirelessly transfer data, the method comprising, at the terminal:
acquiring a user type of a current user (101);
determining (102) if the user type of the current user is a user type for which radiation exposure is to be reduced and, if so, controlling (102, 303) the radiation emitting terminal to reduce a default emission power;
**characterised in that**:
acquiring the user type of the current user comprises:
collecting (301) biological parameters of the current user; and determining (302) the user type of the current user according to the biological parameters;
or,
receiving (402) biological parameters sent from a wearable apparatus, wherein the biological parameters are sent after the wearable apparatus collects the biological parameters of the current user; and determining (403) the user type of the current user according to the biological parameters;
or,
receiving (502) a type signal sent from a wearable apparatus; and acquiring the user type of the current user according to the type signal, wherein the type signal is sent after the wearable apparatus collects biological parameters of the current user and the user type of the current type is determined according to the biological parameters;
wherein the biological parameters include at least one of a heartbeat, a pulse, a sleep record, a motion record, a temperature record and a calorie intake record of a user and the radiation emitting terminal queries the user type of the current user based on a predetermined relationship according to either a value of one biological parameter or values of several biological parameters together;
wherein controlling the radiation emitting terminal to reduce its default emission power comprises:
if the radiation emitting terminal is in a communication state, controlling the radiation emitting terminal to determine the emission power with a condition of satisfying a basic communication quality based on an empirical threshold, wherein the emission power is increased when the signal intensity is lower than the empirical threshold and the emission power is decreased when the signal intensity is higher than the empirical threshold; and
if the radiation emitting terminal is in a standby state, controlling the radiation emitting terminal to determine the emission state with a condition of a minimum radiation value.

2. The method according to claim 1, wherein the method further comprises:
if the radiation emitting terminal is in the communication state and the communication state includes voice communication, optimizing the voice communication in a predetermined manner, wherein the predetermined manner comprises at least one of increasing an audio signal gain, increasing a volume and de-noising.

3. A terminal configured to emit electromagnetic waves to wirelessly transfer data, wherein the terminal comprises:
a type determining module (620) configured to acquire a user type of a current user; and
a radiation reducing module (640) configured to determine whether the user type of the current user is a user type for which for which radiation exposure is to be reduced and, if so, to control the radiation emitting terminal to reduce a default emission power;
**characterized in that**:
the type determining module (620) comprises:
a parameter collecting unit (622) configured to collect biological parameters of the current user; and a type determining unit (623) configured to determine the user type of the current user according to the biological parameters;
or,
a parameter receiving unit (624) configured to receive biological parameters sent from a wearable apparatus, wherein the biological parameters are sent after the wearable apparatus collects the biological parameters of the current user; and the type determining unit (623) being configured to determine the user type of the current user according to the biological parameters;
or,
a type receiving unit (625) configured to receive a type signal sent from a wearable apparatus; and a type acquiring unit (626) configured to acquire the user type of the current user according to the type signal, wherein the type signal is sent after the wearable apparatus collects biological parameters of the current user and the user type of the current user is determined according to the biological parameters;
wherein the biological parameters include at least one of a heartbeat, a pulse, a sleep record, a motion record, a temperature record and a calorie intake record of a user and the radiation emitting terminal is configured to query the user type of the current user based on a predetermined relationship according to either a value of one biological parameter or values of several biological parameters together;
wherein the radiation reducing module comprises a communication reducing unit configured to, when the radiation emitting terminal is in a communication state, control the radiation emitting terminal to determine the emission power with a condition of satisfying a basic communication quality based on an empirical threshold, wherein the emission power is increased when the signal intensity is lower than the empirical threshold and the emission power is decreased when the signal intensity is higher than the empirical threshold;
wherein the radiation reducing module comprises a standby reducing unit, wherein:
the standby reducing unit is configured to, when the radiation emitting terminal is in a standby state, control the radiation emitting terminal to determine the emission power with a condition of a minimum radiation value.

4. The terminal according to claim 3, wherein the apparatus further comprises: a voice optimizing module configured to, when the radiation emitting terminal is in a communication state and the communication state includes voice communication, optimize the voice communication in a predetermined manner, wherein the predetermined manner comprises at least one of increasing an audio signal gain, increasing a volume and denoising.

5. A system for controlling radiation emission from a terminal configured to emit electromagnetic waves to wirelessly transfer data, wherein the system comprises a radiation emitting terminal according to claim 3 and a wearable apparatus, and the wearable apparatus and the radiation emitting terminal are connected to each other through a wired network or a wireless network, wherein
the wearable apparatus is configured to collect (401) the biological parameters of a current user, and send (402) the biological parameters to the radiation emitting terminal of the current user, and
the radiation emitting terminal is further configured to receive the biological parameters sent from the wearable apparatus, determine (403) a user type of the current user according to the biological parameters, and reduce (404) a default emission power when the user type of the current user is a user type for which for which radiation exposure is to be reduced.

6. A system for controlling emission, wherein the system comprises a terminal configured to emit electromagnetic waves according to claim 3 and a wearable apparatus and the wearable apparatus and the radiation emitting terminal are connected to each other through a wired network or a wireless network, wherein
the wearable apparatus is configured to collect (501) the biological parameters of a current user, determine (502) a user type of the current user according to the biological parameters, and send (503) a type signal to the radiation emitting terminal of the current user according to the user type, and
the radiation emitting terminal is configured to receive (504) the type signal sent from the wearable apparatus, acquire the user type of the current user according to the type signal, and reduce (505) a default emission power when the user type of the current user is a user type for which radiation exposure should be reduced.

7. A computer program, which when executed on a processor of a radiation emitting terminal causes it to perform a method according to any one of claims 1 or 2.

## Patentansprüche

1. Verfahren zum Steuern der Strahlungsemission von einem strahlungsemittierenden Terminal, das elektromagnetische Wellen zum drahtlosen Übertragen von Daten benutzt, wobei das Verfahren an dem Terminal Folgendes beinhaltet:
Erfassen eines Benutzertyps eines aktuellen Benutzers (101) ;
Feststellen (102), ob der Benutzertyp des aktuellen Benutzers ein Benutzertyp ist, für den Strahlenbelastung reduziert werden soll, und wenn ja, Steuern (102, 303) des strahlungsemittierenden Terminals zum Reduzieren einer Standard-Emissionsleistung;
**dadurch gekennzeichnet, dass**:
das Erfassen des Benutzertyps des aktuellen Benutzers Folgendes beinhaltet:
Sammeln (301) von biologischen Parametern des aktuellen Benutzers; und Bestimmen (302) des Benutzertyps des aktuellen Benutzers gemäß den biologischen Parametern; oder
Empfangen (402) von von einer tragbaren Vorrichtung gesendeten biologischen Parametern, wobei die biologischen Parameter gesendet werden, nachdem die tragbare Vorrichtung die biologischen Parameter des aktuellen Benutzers gesammelt hat; und Bestimmen (403) des Benutzertyps des aktuellen Benutzers gemäß den biologischen Parametern; oder
Empfangen (502) eines von einer tragbaren Vorrichtung gesendeten Typensignals; und Erfassen des Benutzertyps des aktuellen Benutzers gemäß dem Typensignal, wobei das Typensignal gesendet wird, nachdem die tragbare Vorrichtung biologische Parameter des aktuellen Benutzers gesammelt hat und der Benutzertyp des aktuellen Benutzers gemäß den biologischen Parametern bestimmt wird;
wobei die biologischen Parameter wenigstens eines aus Herzschlag, Puls, Schlafprotokoll, Bewegungsprotokoll, Temperaturprotokoll und Kalorienaufnahmeprotokoll eines Benutzers beinhalten und das strahlungsemittierende Terminal den Benutzertyp des aktuellen Benutzers auf der Basis einer vorbestimmten Beziehung entweder gemäß einem Wert eines biologischen Parameters oder von Werten von mehreren biologischen Parametern zusammen abfragt;
wobei das Steuern des strahlungsemittierenden Terminals zum Reduzieren seiner Standard-Emissionsleistung Folgendes beinhaltet:
Steuern, wenn das strahlungsemittierende Terminal in einem Kommunikationszustand ist, des strahlungsemittierenden Terminals zum Bestimmen der Emissionsleistung unter einer Bedingung des Erfüllens einer Grundkommunikationsqualität auf der Basis einer empirischen Schwelle, wobei die Emissionsleistung erhöht wird, wenn die Signalintensität niedriger ist als die empirische Schwelle, und die Emissionsleistung verringert wird, wenn die Signalintensität höher ist als die empirische Schwelle; und
Steuern, wenn das strahlungsemittierende Terminal in einem Standby-Zustand ist, des strahlungsemittierenden Terminals zum Bestimmen des Emissionszustands unter einer Bedingung eines minimalen Strahlungswertes.

2. Verfahren nach Anspruch 1, wobei das Verfahren ferner Folgendes beinhaltet:
Optimieren, wenn das strahlungsemittierende Terminal im Kommunikationszustand ist und der Kommunikationszustand Sprachkommunikation beinhaltet, der Sprachkommunikation auf eine vorbestimmte Weise, wobei die vorbestimmte Weise wenigstens eines aus Erhöhen einer Audiosignalverstärkung, Erhöhen einer Lautstärke und Geräuschreduzierung beinhaltet.

3. Terminal, konfiguriert zum Emittieren von elektromagnetischen Wellen zum drahtlosen Übertragen von Daten, wobei das Terminal Folgendes umfasst:
ein Typenbestimmungsmodul (620), konfiguriert zum Erfassen eines Benutzertyps eines aktuellen Benutzers; und
ein Strahlungsreduktionsmodul (640), konfiguriert zum Feststellen (102), ob der Benutzertyp des aktuellen Benutzers ein Benutzertyp ist, für den Strahlenbelastung reduziert werden soll, und wenn ja, zum Steuern des strahlungsemittierenden Terminals zum Reduzieren einer Standard-Emissionsleistung;
**dadurch gekennzeichnet, dass**:
das Typenbestimmungsmodul (620) Folgendes umfasst:
eine Parametersammeleinheit (622), konfiguriert zum Sammeln von biologischen Parametern des aktuellen Benutzers; und eine Typenbestimmungseinheit (623), konfiguriert zum Bestimmen des Benutzertyps des aktuellen Benutzers gemäß den biologischen Parametern; oder
eine Parameterempfangseinheit (624), konfiguriert zum Empfangen von von einer tragbaren Vorrichtung gesendeten biologischen Parametern, wobei die biologischen Parameter gesendet werden, nachdem die tragbare Vorrichtung die biologischen Parameter des aktuellen Benutzers gesammelt hat; und wobei die Typenbestimmungseinheit (623) zum Bestimmen des Benutzertyps des aktuellen Benutzers gemäß den biologischen Parametern konfiguriert ist; oder
eine Typenempfangseinheit (625), konfiguriert zum Empfangen eines von einer tragbaren Vorrichtung gesendeten Typensignals; und eine Typenerfassungseinheit (626), konfiguriert zum Erfassen des Benutzertyps des aktuellen Benutzers gemäß dem Typensignal, wobei das Typensignal gesendet wird, nachdem die tragbare Vorrichtung biologische Parameter des aktuellen Benutzers gesammelt hat und der Benutzertyp des aktuellen Benutzers gemäß den biologischen Parametern bestimmt wurde;
wobei die biologischen Parameter wenigstens eines aus Herzschlag, Puls, Schlafprotokoll, Bewegungsprotokoll, Temperaturprotokoll und Kalorienaufnahmeprotokoll eines Benutzers beinhalten und das strahlungsemittierende Terminal zum Abfragen des Benutzertyps des aktuellen Benutzers auf der Basis einer vorbestimmten Beziehung entweder gemäß einem Wert von einem biologischen Parameter oder von Werten von mehreren biologischen Parametern zusammen konfiguriert ist;
wobei das Strahlungsreduktionsmodul eine Kommunikationsreduktionseinheit umfasst, konfiguriert zum Steuern, wenn das strahlungsemittierende Terminal in einem Kommunikationszustand ist, des strahlungsemittierenden Terminals zum Bestimmen der Emissionsleistung unter einer Bedingung des Erfüllens einer Grundkommunikationsqualität auf der Basis einer empirischen Schwelle, wobei die Emissionsleistung erhöht wird, wenn die Signalintensität niedriger ist als die empirische Schwelle, und die Emissionsleistung verringert wird, wenn die Signalintensität höher ist als die empirische Schwelle;
wobei das Strahlungsreduktionsmodul eine Standby-Reduktionseinheit umfasst, wobei:
die Standby-Reduktionseinheit konfiguriert ist zum Steuern, wenn das strahlungsemittierende Terminal in einem Standby-Zustand ist, des strahlungsemittierenden Terminals zum Bestimmen der Emissionsleistung unter einer Bedingung eines minimalen Strahlungswertes.

4. Terminal nach Anspruch 3, wobei die Vorrichtung ferner Folgendes umfasst:
ein Sprachoptimierungsmodul, konfiguriert zum Optimieren, wenn das strahlungsemittierende Terminal in einem Kommunikationszustand ist und der Kommunikationszustand Sprachkommunikation beinhaltet, der Sprachkommunikation auf eine vorbestimmte Weise, wobei die vorbestimmte Weise wenigstens eines aus Erhöhen einer Audiosignalverstärkung, Erhöhen einer Lautstärke und Rauschunterdrückung beinhaltet.

5. System zum Steuern von Strahlungsemission von einem Terminal, konfiguriert zum Emittieren von elektromagnetischen Wellen zum drahtlosen Übertragen von Daten, wobei das System ein strahlungsemittierendes Terminal nach Anspruch 3 und eine tragbare Vorrichtung umfasst und die tragbare Vorrichtung und das strahlungsemittierende Terminal durch ein verdrahtetes Netzwerk oder ein drahtloses Netzwerk miteinander verbunden sind, wobei
die tragbare Vorrichtung zum Sammeln (401) der biologischen Parameter eines aktuellen Benutzers und zum Senden (402) der biologischen Parameter zu dem strahlungsemittierenden Terminal des aktuellen Benutzers konfiguriert ist, und
das strahlungsemittierende Terminal ferner zum Empfangen der von der tragbaren Vorrichtung gesendeten biologischen Parameter, zum Bestimmen (403) eines Benutzertyps des aktuellen Benutzers gemäß den biologischen Parametern und zum Reduzieren (404) einer Standard-Emissionsleistung konfiguriert ist, wenn der Benutzertyp des aktuellen Benutzers ein Benutzertyp ist, für den Strahlenbelastung reduziert werden soll.

6. System zum Steuern von Emissionen, wobei das System ein Terminal umfasst, konfiguriert zum Emittieren von elektromagnetischen Wellen nach Anspruch 3, und eine tragbare Vorrichtung, und die tragbare Vorrichtung und das strahlungsemittierende Terminal durch ein verdrahtetes Netzwerk oder ein drahtloses Netzwerk miteinander verbunden sind, wobei
die tragbare Vorrichtung zum Sammeln (501) der biologischen Parameter eines aktuellen Benutzers, zum Bestimmen (502) eines Benutzertyps des aktuellen Benutzers gemäß den biologischen Parametern und zum Senden (503) eines Typensignals zu dem strahlungsemittierenden Terminal des aktuellen Benutzers gemäß dem Benutzertyp konfiguriert ist,
und
das strahlungsemittierende Terminal zum Empfangen (504) des von der tragbaren Vorrichtung gesendeten Typensignals, zum Empfangen des Benutzertyps des aktuellen Benutzers gemäß dem Typensignal und zum Reduzieren (505) einer Standard-Emissionsleistung konfiguriert ist, wenn der Benutzertyp des aktuellen Benutzers ein Benutzertyp ist, für den Strahlenbelastung reduziert werden soll.

7. Computerprogramm, das bei Ausführung auf einem Prozessor eines strahlungsemittierenden Terminals bewirkt, dass er ein Verfahren nach Anspruch 1 oder 2 durchführt.

## Revendications

1. Procédé de régulation de l'émission de rayonnement d'un terminal émetteur de rayonnement qui utilise des ondes électromagnétiques pour transférer en mode sans fil des données, le procédé comprenant, au niveau du terminal :
l'acquisition d'un type d'utilisateur d'un utilisateur actuel (101) ;
la détermination (102) que le type d'utilisateur de l'utilisateur actuel est ou non un type d'utilisateur pour lequel l'exposition au rayonnement doit être réduite et, dans l'affirmative, la régulation (102, 303) du terminal émetteur de rayonnement pour réduire une puissance d'émission par défaut ;
**caractérisé en ce que** :
l'acquisition du type d'utilisateur de l'utilisateur actuel comprend :
la collecte (301) de paramètres biologiques de l'utilisateur actuel ; et la détermination (302) du type d'utilisateur de l'utilisateur actuel conformément aux paramètres biologiques ;
ou,
la réception (402) de paramètres biologiques envoyés par un appareil connecté, les paramètres biologiques étant envoyés après que l'appareil connecté collecte les paramètres biologiques de l'utilisateur actuel ; et la détermination (403) du type d'utilisateur de l'utilisateur actuel conformément aux paramètres biologiques;
ou,
la réception (502) d'un signal de type envoyé par un appareil connecté ; et l'acquisition du type d'utilisateur de l'utilisateur actuel conformément au signal de type, le signal de type étant envoyé après que l'appareil connecté collecte des paramètres biologiques de l'utilisateur actuel et le type d'utilisateur du type actuel est déterminé conformément aux paramètres biologiques ;
dans lequel les paramètres biologiques comportent au moins un d'un rythme cardiaque, d'un pouls, d'un enregistrement de sommeil, d'un enregistrement de mouvement, d'un enregistrement de température et d'un enregistrement d'apport calorique d'un utilisateur et le terminal émetteur de rayonnement interroge le type d'utilisateur de l'utilisateur actuel en fonction d'une relation prédéterminée conformément à soit une valeur d'un paramètre biologique, soit des valeurs de plusieurs paramètres biologiques ensemble ;
dans lequel la régulation du terminal émetteur de rayonnement pour réduire sa puissance d'émission par défaut comprend :
si le terminal émetteur de rayonnement est dans un état de communication, la régulation du terminal émetteur de rayonnement pour déterminer la puissance d'émission avec une condition de satisfaction d'une qualité de communication de base basée sur un seuil empirique, dans lequel la puissance d'émission est augmentée quand l'intensité de signal est inférieure au seuil empirique et la puissance d'émission est réduite quand l'intensité de signal est supérieure au seuil empirique
; et
si le terminal émetteur de rayonnement est dans un état de veille, la régulation du terminal émetteur de rayonnement pour déterminer l'état d'émission avec une condition de valeur de rayonnement minimum.

2. Procédé selon la revendication 1, le procédé comprenant en outre :
si le terminal émetteur de rayonnement est dans l'état de communication et l'état de communication comporte une communication vocale, l'optimisation de la communication vocale d'une manière prédéterminée, la manière prédéterminée comprenant au moins une d'une augmentation d'un gain de signal audio, d'une augmentation d'un volume et d'une suppression de bruit.

3. Terminal configuré pour émettre des ondes électromagnétiques afin de transférer en mode sans fil des données, le terminal comprenant :
un module de détermination de type (620) configuré pour acquérir un type d'utilisateur d'un utilisateur actuel ; et
un module de réduction de rayonnement (640) configuré pour déterminer que le type d'utilisateur de l'utilisateur actuel est ou non un type d'utilisateur pour lequel l'exposition au rayonnement doit être réduite et, dans l'affirmative, réguler le terminal émetteur de rayonnement pour réduire une puissance d'émission par défaut ;
**caractérisé en ce que** :
le module de détermination de type (620) comprend :
une unité de collecte de paramètres (622) configurée pour collecter des paramètres biologiques de l'utilisateur actuel ; et une unité de détermination de type (623) configurée pour déterminer le type d'utilisateur de l'utilisateur actuel conformément aux paramètres biologiques ;
ou,
une unité de réception de paramètres (624) configurée pour recevoir des paramètres biologiques envoyés par un appareil connecté, les paramètres biologiques étant envoyés après que l'appareil connecté collecte les paramètres biologiques de l'utilisateur actuel ; et l'unité de détermination de type (623) étant configurée pour déterminer le type d'utilisateur de l'utilisateur actuel conformément aux paramètres biologiques ;
ou,
une unité de réception de type (625) configurée pour recevoir un type de signal envoyé par un appareil connecté ; et une unité d'acquisition de type (626) configurée pour acquérir le type d'utilisateur de l'utilisateur actuel conformément au signal de type, le signal de type étant envoyé après que l'appareil connecté collecte des paramètres biologiques de l'utilisateur actuel et le type d'utilisateur du type actuel est déterminé conformément aux paramètres biologiques ;
dans lequel les paramètres biologiques comportent au moins un d'un rythme cardiaque, d'un pouls, d'un enregistrement de sommeil, d'un enregistrement de mouvement, d'un enregistrement de température et d'un enregistrement d'apport calorique d'un utilisateur et le terminal émetteur de rayonnement est configuré pour interroger le type d'utilisateur de l'utilisateur actuel en fonction d'une relation prédéterminée conformément à soit une valeur d'un paramètre biologique, soit des valeurs de plusieurs paramètres biologiques ensemble ;
dans lequel le module de réduction de rayonnement comprend une unité de réduction de communication configurée pour, quand le terminal émetteur de rayonnement est dans un état de communication, réguler le terminal émetteur de rayonnement pour déterminer la puissance d'émission avec une condition de satisfaction d'une qualité de communication de base basée sur un seuil empirique, dans lequel la puissance d'émission est augmentée quand l'intensité de signal est inférieure au seuil empirique et la puissance d'émission est réduite quand l'intensité de signal est supérieure au seuil empirique ;
dans lequel le module de réduction de rayonnement comprend une unité de réduction en veille, dans lequel :
l'unité de réduction en veille est configurée pour, quand le terminal émetteur de rayonnement est dans un état de veille, réguler le terminal émetteur de rayonnement pour déterminer l'état d'émission avec une condition de valeur de rayonnement minimum.

4. Terminal selon la revendication 3, dans lequel l'appareil comprend en outre :
un module d'optimisation vocale configuré pour, quand le terminal émetteur de rayonnement est dans un état de communication et l'état de communication comporte une communication vocale, optimiser la communication vocale d'une manière prédéterminée, la manière prédéterminée comprenant au moins une d'une augmentation d'un gain de signal audio, d'une augmentation d'un volume et d'une suppression de bruit.

5. Système de régulation d'une émission de rayonnement d'un terminal configuré pour émettre des ondes électromagnétiques afin de transférer en mode sans fil des données, le système comprenant un terminal émetteur de rayonnement selon la revendication 3 et un appareil connecté, et l'appareil connecté et le terminal émetteur de rayonnement sont connectés l'un à l'autre par le biais d'un réseau filaire ou d'un réseau sans fil, dans lequel
l'appareil connecté est configuré pour collecter (401) les paramètres biologiques d'un utilisateur actuel, et envoyer (402) les paramètres biologiques au terminal émetteur de rayonnement de l'utilisateur actuel, et
le terminal émetteur de rayonnement est configuré en outre pour recevoir les paramètres biologiques envoyés par l'appareil connecté, déterminer (403) un type d'utilisateur de l'utilisateur actuel conformément aux paramètres biologiques, et réduire (404) une puissance d'émission par défaut quand le type d'utilisateur de l'utilisateur actuel est un type d'utilisateur pour lequel l'exposition au rayonnement doit être réduite.

6. Système de régulation d'émission, le système comprenant un terminal configuré pour émettre des ondes électromagnétiques selon la revendication 3 et un appareil connecté et l'appareil connecté et le terminal émetteur de rayonnement sont connectés l'un à l'autre par le biais d'un réseau filaire ou d'un réseau sans fil, dans lequel
l'appareil connecté est configuré pour collecter (501) les paramètres biologiques d'un utilisateur actuel, déterminer (502) un type d'utilisateur de l'utilisateur actuel conformément aux paramètres biologiques, et envoyer (503) un type de signal au terminal émetteur de rayonnement de l'utilisateur actuel conformément au type d'utilisateur, et
le terminal émetteur de rayonnement est configuré pour recevoir (504) le signal de type envoyé par l'appareil connecté, acquérir le type d'utilisateur de l'utilisateur actuel conformément au signal de type, et réduire (505) une puissance d'émission par défaut quand le type d'utilisateur de l'utilisateur actuel est un type d'utilisateur pour lequel l'exposition au rayonnement doit être réduite.

7. Programme informatique, lequel, à son exécution sur un processeur d'un terminal émetteur de rayonnement, amène celui-ci à réaliser un procédé selon l'une quelconque des revendications 1 ou 2.
